# EUROPEAN PATENT SPECIFICATION

(11) **EP 2 482 740 B1**
(45) Date of publication and mention of the grant of the patent: **21.11.2018**
(21) Application number: 10820972.7
(22) Date of filing: 13.07.2010
(51) Int. Cl.: A61B 17/22, A61B 17/3207

(54) **ROTATIONAL ATHERECTOMY DEVICE WITH FRICTIONAL CLUTCH HAVING MAGNETIC NORMAL FORCE**
ROTIERENDE ATHEREKTOMIEVORRICHTUNG MIT REIBUNGSKUPPLUNG MIT NORMALER MAGNETKRAFT
DISPOSITIF D'ATHÉRECTOMIE ROTATIF AVEC EMBRAYAGE À FRICTION AYANT UNE FORCE NORMALE MAGNÉTIQUE

(30) Priority: 29.09.2009 US 568939
(43) Date of publication of application: 08.08.2012
(73) Proprietor: Cardiovascular Systems, Inc., St. Paul, MN 55112 (US)
(72) Inventor: GRUBAC, Vladimir, Brookly Park MN 55428 (US); DOBROVOLNY, Walter, John, St. Paul MN 55117 (US); SCHOENLE, Victor, Leo, Greenfield MN 55357 (US)
(74) Representative: Viering, Jentschura & Partner mbB Patent- und Rechtsanwälte
(86) International application number: PCT/US2010/041787
(87) International publication number: WO 2011/041010

(56) References cited:
- WO-A2-01/78605
- US-A- 5 569 179
- US-A- 5 569 179
- US-B1- 6 517 528

## Description

### CROSS-REFERENCE TO RELATED APPLICATIONS

Not Applicable

### STATEMENT REGARDING FEDERALLY SPONSORED RESEARCH OR DEVELOPMENT

Not Applicable

### BACKGROUND OF THE INVENTION

### FIELD OF THE INVENTION

The invention relates to devices for removing tissue from body passageways, such as removal of atherosclerotic plaque from arteries, utilizing a rotational atherectomy device. In particular, the invention relates to improvements in a rotational atherectomy device having a frictional clutch that has a magnetically-induced normal force.

### DESCRIPTION OF THE RELATED ART

Atherectomy is a non-surgical procedure to open blocked coronary arteries or vein grafts by using a device on the end of a catheter to cut or shave away atherosclerotic plaque (a deposit of fat and other substances that accumulate in the lining of the artery wall). For the purposes of this application, the term "abrading" is used to describe the grinding and/or scraping action of such an atherectomy head.

Atherectomy is performed to restore the flow of oxygen-rich blood to the heart, to relieve chest pain, and to prevent heart attacks. It may be done on patients with chest pain who have not responded to other medical therapy and on certain of those who are candidates for balloon angioplasty (a surgical procedure in which a balloon catheter is used to flatten plaque against an artery wall) or coronary artery bypass graft surgery. It is sometimes performed to remove plaque that has built up after a coronary artery bypass graft surgery.

Atherectomy uses a rotating shaver or other device placed on the distal end of a catheter to slice away or destroy plaque. At the beginning of the procedure, medications to control blood pressure, dilate the coronary arteries, and prevent blood clots are administered. The patient is awake but sedated. The catheter is inserted into an artery in the groin, leg, or arm, and threaded through the blood vessels into the blocked coronary artery. The cutting head is positioned against the plaque and activated, and the plaque is ground up or suctioned out.

The types of atherectomy are rotational, directional, and transluminal extraction. Rotational atherectomy uses a high speed rotating shaver to grind up plaque. Directional atherectomy was the first type approved, but is no longer commonly used; it scrapes plaque into an opening in one side of the catheter. Transluminal extraction coronary atherectomy uses a device that cuts plaque off vessel walls and vacuums it into a bottle. It is used to clear bypass grafts.

Performed in a cardiac catheterization lab, atherectomy is also called removal of plaque from the coronary arteries. It can be used instead of, or along with, balloon angioplasty.

Several devices have been disclosed that perform rotational atherectomy. For instance, U.S. Patent No. 5,360,432, issued on November 1, 1994 to Leonid Shturman, and titled "Abrasive drive shaft device for directional rotational atherectomy" discloses an abrasive drive shaft atherectomy device for removing stenotic tissue from an artery. The device includes a rotational atherectomy apparatus having a flexible, elongated drive shaft having a central lumen and a segment, near its distal end, coated with an abrasive material to define an abrasive segment. At sufficiently high rotational speeds, the abrasive segment expands radially, and can sweep out an abrading diameter that is larger than its rest diameter. In this manner, the atherectomy device may remove a blockage that is larger than the catheter itself. Use of an expandable head is an improvement over atherectomy devices that use non-expandable heads; such non-expandable devices typically require removal of particular blockages in stages, with each stage using a differently-sized head.

U.S. Pat. No. 5,314,438 (Shturman) shows another atherectomy device having a rotatable drive shaft with a section of the drive shaft having an enlarged diameter, at least a segment of this enlarged diameter section being covered with an abrasive material to define an abrasive segment of the drive shaft. When rotated at high speeds, the abrasive segment is capable of removing stenotic tissue from an artery.

A typical atherectomy device includes a single-use disposable portion, which can be attached and detached from a non-disposable control unit (also referred to as a controller). The disposable portion includes elements that are exposed to saline and to the bodily fluids of the patient, such as a handle, a catheter, a rotatable drive shaft, and an abrasive head. The handle includes a turbine that rotates the drive shaft, and a knob that can longitudinally advance and retract the drive shaft along the catheter. Often, the device has a foot switch that activates the handle.

Typical known atherectomy devices use pneumatic power to drive the drive shaft, with the controller managing the amount of compressed air that is delivered to the turbine in the handle. The compressed air spins the turbine that, in turn, spins the drive shaft, and spins an abrasive crown attached to the drive shaft. Orbiting motion of the crown enlarges and widens the channel opening of a restricted or blocked vascular vessel.

There is currently a great deal of effort devoted to incorporating other types of rotational actuators into the atherectomy devices, primarily to replace the need for a source of compressed air. A motor requires a way limit the torque delivered to the drive shaft. For instance, if the distal end of the drive shaft encounters an obstacle and gets stuck (i.e., stops rotating), it is preferable that the torque delivered to the drive shaft be limited, so that the drive shaft does not wind up excessively and abruptly release. Such a sudden release of energy may result in damage to the patient or the device, and should be avoided.

Accordingly, there exists a need for a clutch between the motor and the drive shaft in a rotational atherectomy device.

WO 01/78605 A2 discloses a system whereby a driven member is inserted into a magnetic sleeve attached to the driver member.

### BRIEF SUMMARY OF THE INVENTION

Claim 1 defines the invention and the dependent claims disclose the preferred embodiments, An embodiment is a rotational atherectomy system, comprising: an elongated, rotatable, flexible drive shaft having a distal end for insertion into a vasculature of a patient and having a proximal end opposite the distal end remaining outside the vasculature of the patient; a motor for rotating the drive shaft; and a clutch having a characteristic threshold torque, comprising: a motor plate rotationally connected to the motor; and a drive shaft plate rotationally connected to the drive shaft, the motor plate and the drive shaft plate being parallel and coaxial, being disposed directly longitudinally adjacent to each other, and being held together longitudinally by a magnetic attractive force between the motor plate and the drive shaft plate. Torques between the motor and the drive shaft less than the threshold torque are transmitted completely between the motor plate and the drive shaft plate, which remain held together rotationally by static friction. Torques between the motor and the drive shaft greater than the threshold torque cause the motor plate and the drive shaft plate to slip rotationally past each other. Torques between the motor and the drive shaft greater than the threshold torque cause a residual torque to be transmitted between the motor and the drive shaft, the residual torque being less than the threshold torque and being determined by a kinetic coefficient of friction between the motor plate and the drive shaft plate.

An other embodiment is a rotational atherectomy system, comprising: an elongated, rotatable, flexible drive shaft having a distal end for insertion into a vasculature of a patient and having a proximal end opposite the distal end remaining outside the vasculature of the patient; a motor for rotating the drive shaft; and a clutch for transmitting relative torques between the motor and the proximal end of the drive shaft, the clutch comprising: a motor plate rotatably connected to the motor; and a drive shaft plate rotatably connected to the drive shaft and directly longitudinally adjacent to and parallel to the motor plate. The motor plate and the drive shaft plate are magnetically attracted to each other, the magnetic attraction forming a normal force. The normal force holds together the motor plate and the drive shaft plate for relative torques less than a threshold torque, the threshold torque being directly proportional to the normal force. The motor plate and the drive shaft plate slip past each other for relative torques greater than the threshold torque. The motor plate transmits a residual torque to the drive shaft plate when the motor plate and the drive shaft plate slip past each other, the residual torque being directly proportional to the normal force and being less than the threshold torque.

Another embodiment is a rotational atherectomy system, comprising: an elongated, rotatable, flexible drive shaft having a distal end for insertion into a vasculature of a patient and having a proximal end opposite the distal end remaining outside the vasculature of the patient; a motor for rotating the drive shaft; and a clutch for transmitting torque between the motor and the proximal end of the drive shaft, the clutch fully transmitting torque less than a threshold torque, the clutch transmitting a residual torque for torque greater than the threshold torque. The residual torque is less than the threshold torque.

### BRIEF DESCRIPTION OF THE SEVERAL VIEWS OF THE DRAWINGS

Figure 1 is a perspective view of a known rotational atherectomy device.
Figure 2 is a block diagram of the motor, the drive shaft and the clutch that mechanically couples them together.
Figure 3 is a schematic drawing of the clutch of Figure 2.
Figure 4 is a plot of rotational speed of the drive shaft and torque at the distal end of the drive shaft, for a typical procedure.
Figure 5 is a plot of the torque transmitted to the proximal end of the drive shaft, versus the torque of the motor.
Figure 6 is a plot of torque at the distal end of the drive shaft versus time for a distal-end-stopping event, for a known gas turbine system.
Figure 7 is a plot of torque at the distal end of the drive shaft versus time for a distal-end-stopping event, for the present motor-driven system with the clutch of Figure 3.

### DETAILED DESCRIPTION OF THE INVENTION

An atherectomy device is disclosed, with a clutch between the motor and the drive shaft. The clutch includes two plates that rely on friction to transmit torque from one plate to the other. The clutch has an attractive magnetic normal force that holds the plates together. For relatively low torques, as is the case during normal use, a static frictional torque holds the plates together, and the plates spin together without slipping. For relatively high torques, as occurs when the distal end of the drive shaft encounters an obstacle and stops abruptly, the high torque exceeds the maximum possible static frictional torque, and the plates slip. When slipping, the plates transmit a kinetic frictional torque that is low enough to avoid damage to the patient or to the atherectomy device. In some cases, the torque levels associated with a stoppage of the drive shaft distal end are chosen to mimic those of a known atherectomy device, in which a gas-driven turbine is clutchlessly attached to the drive shaft.

The preceding paragraph is merely a summary, and should not be construed as limiting in any way. A more detailed description follows.

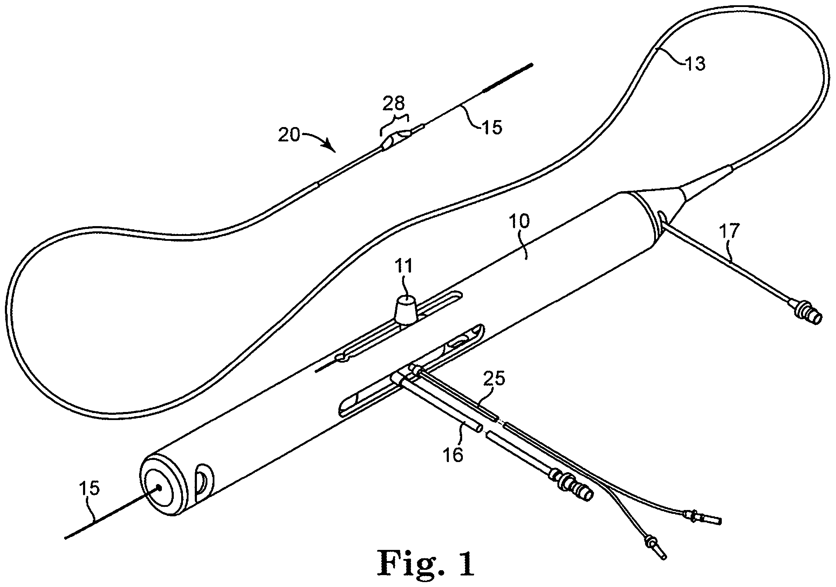

Figure 1 is a schematic drawing of a typical rotational atherectomy device. The device includes a handle portion 10, an elongated, flexible drive shaft 20 having an eccentric enlarged abrading head 28, and an elongated catheter 13 extending distally from the handle portion 10. The drive shaft 20 is constructed from helically coiled wire as is known in the art and the abrading head 28 is fixedly attached thereto. The catheter 13 has a lumen in which most of the length of the drive shaft 20 is disposed, except for the enlarged abrading head 28 and a short section distal to the enlarged abrading head 28. The drive shaft 20 also contains an inner lumen, permitting the drive shaft 20 to be advanced and rotated over a guide wire 15. A fluid supply line 17 may be provided for introducing a cooling and lubricating solution (typically saline or another biocompatible fluid) into the catheter 13.

The handle 10 desirably contains a turbine (or similar rotational drive mechanism) for rotating the drive shaft 20 at high speeds. The handle 10 typically may be connected to a power source, such as compressed air delivered through a tube 16. A pair of fiber optic cables 25, alternatively a single fiber optic cable may be used, may also be provided for monitoring the speed of rotation of the turbine and drive shaft 20. Details regarding such handles and associated instrumentation are well known in the industry, and are described, e.g., in U.S. Pat. No. 5,314,407, issued to Auth. The handle 10 also desirably includes a control knob 11 for advancing and retracting the turbine and drive shaft 20 with respect to the catheter 13 and the body of the handle.

The abrasive element 28 in Figure 1 is an eccentric solid crown, attached to the drive shaft 20 near the distal end of the drive shaft 20. The term "eccentric" is used herein to denote that the center of mass of the crown is laterally displaced away from the rotational axis of the drive shaft 20. As the drive shaft rotates rapidly, the displaced center of mass of the crown causes the drive shaft to flex radially outward in the vicinity of the crown as it spins, so that the crown may abrade over a larger diameter than its own rest diameter. Eccentric solid crowns are disclosed in detail in, for example, U.S. Patent Application No. 11/761,128, filed on June 11, 2007 to Thatcher et al. under the title, "Eccentric abrading head for high-speed rotational atherectomy devices", published on December 11, 2008 as U.S. Patent Application Publication No. US2008/0306498.

There is currently an effort to replace the gas-driven turbine of the known atherectomy device with an electric motor. Such a motor has different mechanical characteristics than the turbine, such as an increased rotational inertia. The present application is directed mainly to a clutch that connects a motor to the drive shaft. Such a clutch can limit the torque delivered by the motor, so that if the distal end of the drive shaft encounters an obstacle and suddenly stops rotating, the clutch will prevent a damaging amount of torque from being delivered to the drive shaft. Aside from the motor, many or all of the other elements of the known atherectomy device of Figure 1 may be used with the present disclosed head design, including the catheter 13, the guide wire 15, the control knob 11 on the handle 10, the helically coiled drive shaft 20 and the eccentric solid crown 28.

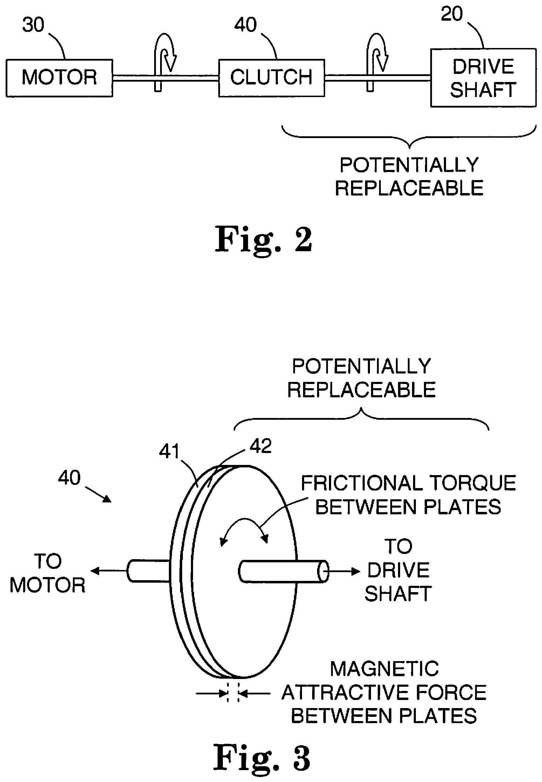

Figure 2 is a block diagram of the motor 30, the drive shaft 20 and the clutch 40 that mechanically couples them together. In this figure and those that follow, the "motor" may be an electric motor, a gas-driven turbine, or any suitable device that generates a controllable amount of rotation. During normal use, the clutch 40 is engaged, and the rotation produced by the motor 30 is passed directly on to the drive shaft 20. In the event that the distal end of the drive shaft 20 becomes caught or encounters a blockage that suddenly stops its rotation, the clutch disengages, so that the motor 30 does not continue to rotate the proximal end of the drive shaft. Such a continued rotation would excessively wind up the drive shaft, and the torques associated with such a winding could potentially damage the blood vessel of the patient or the atherectomy device itself, which are both undesirable outcomes.

Additionally, the clutch may provide a convenient interface between the drive shaft, which is typically a replaceable or disposable element, and the motor, which is typically used repeatedly.

Figure 3 is a schematic drawing of the clutch 40 of Figure 2. The clutch 40 includes two plates, 41 and 42, held together by an attractive magnetic force. The plates 41, 42 are attached to spindles that rotationally couple them to the motor 30 and drive shaft 20, respectively.

During normal operation, including spin-up, constant rotational speeds, and spin-down, the difference in torque between the motor and the proximal end of the drive shaft is relatively small. For these small torque differences, the magnetic attractive force is sufficient to hold the plates 41 and 42 together, and the proximal end of the drive shaft is spun along with the motor.

If the distal end of the drive shaft encounters an obstacle and is suddenly stopped from rotating, the torque difference between the motor and the proximal end of the drive shaft increases rapidly and eventually exceeds the static frictional torque that holds the plates together. When this happens, the plates slip rotationally with respect to each other, and transmit a kinetic frictional torque from one to the other as they slip. A detailed discussion of these frictional effects follows below.

Note that torque is the rotational analog of the quantity, force. Torque produces a change in angular momentum, much like linear force produces a change in linear momentum. Because the rotational inertia of the device components remains roughly constant throughout their operation, a non-zero torque therefore produces a change in rotational speed.

Note also that the two plates 41 and 42, which are held together magnetically, may provide a convenient interface for replacement. For instance, after a procedure has been performed, the drive shaft and associated mechanical parts may be removed by detaching the magnetically-attracted plates 41 and 42. Plate 42 is disposed of, along with the drive shaft, while plate 41 remains with the motor unit and may be used repeatedly.

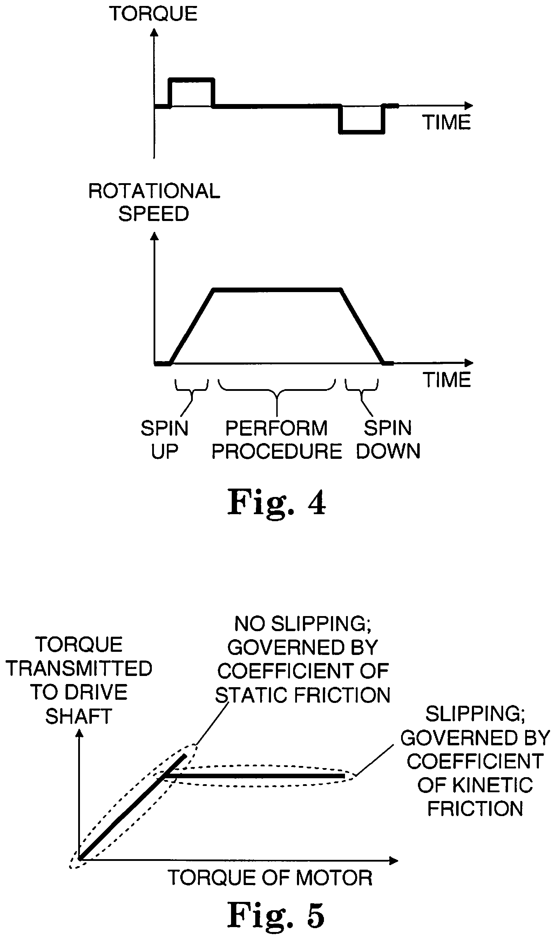

Figure 4 is a plot of rotational speed of the drive shaft and torque at the distal end of the drive shaft, for a typical procedure. Initially, the drive shaft is at rest and there are no net torques present. During the "spin-up" phase, the motor applies a non-zero torque to the proximal end of the drive shaft, and the rotational speed of the drive shaft increases. Once a desired rotational speed is reached, the torque of the motor is reduced to keep the drive shaft at a constant rotational speed. Note that the actual torque applied by the motor to the proximal end of the drive shaft may be small but non-zero, in order to overcome the effects of friction between the proximal and distal ends of the drive shaft. The plot shows the torque at the distal end of the drive shaft, which is truly zero when the distal end of the drive shaft rotates at a constant rotational speed. During the "spin-down" phase, the motor applies a non-zero torque in the opposite direction to reduce the rotational speed of the drive shaft to zero.

The typical torque levels shown in Figure 4, which commonly occur during use, are usually below a threshold at which the plates 41, 42 in the clutch 40 begin to slip. During normal use, the clutch remains engaged, and the static frictional force between the plates holds the plates together. It is desired that the plates slip, and the clutch disengages, only during an atypical event, such as when the distal end of the drive shaft becomes stuck and stops rotating. However, it is possible that the plates may slip during spin-up and/or spin-down, due to the spin-up and/or spin-down torques exceeding the threshold.

At this point, it is instructive to review the physics of frictional forces, in order to better understand when the clutch plates hold together, and when they slip.

Consider for a moment two linear plates, rather than two rotating plates as in the true clutch of Figure 3. The linear plates are held together by a normal force that can be generated magnetically, as is the case of the clutch of Figure 3, or can be generated externally. For relatively small forces parallel to the contact surfaces, the plates hold together. In other words, if one pushes gently on one plate, parallel to the contact surfaces, the other plate holds with it and there is no slippage. For relatively large forces parallel to the contact surface, such as a strike with a hammer, the plates no longer hold together, and slip past each other along the contact surface.

The threshold at which slippage begins to occur is given by the product of the normal force (i.e., the force holding the plates together, generated magnetically or otherwise) and a coefficient of static friction. The coefficient of static friction is a dimensionless quantity that is typically less than one. For forces less than this threshold, the plates hold together. For forces greater than this threshold, the plates slip.

As an example, consider the interface between a rubber tire and a road surface. For a small normal force, as is the case when the tire is simply resting on the road under the effects of its own gravitational weight, it is easy to drag the tire along the road surface. For a large normal force, as is the case when the tire supports the weight of a car, it is quite difficult to overcome the frictional forces that keep the tire in contact with the road. In practice, skidding only occurs for large forces, such as slamming on the brakes during driving conditions.

From this example, we may state a first general principle for our clutch: the normal force (i.e., the magnetically-generated force that attracts the plates to each other) determines the threshold at which slipping between the plates begins to occur.

Such a normal force is controllable at the design phase of the clutch, and may be controlled by the lateral distribution of magnetic materials in the plates, as well as the longitudinal distribution of those materials. For instance, the normal force decreases as the longitudinal spacing between the magnetic particles increases; such spacing can be achieved in many ways, such as by coating the magnetic particles with a non-magnetic layer.

Returning to the example of the two linear plates, consider now the case when the plates are already slipping past each other. There is a resisting force generated at the contact surfaces, which would slow down and eventually stop the slipping motion, if no other forces were at work. Likewise, if one were to push of the sliding plates parallel to the surfaces with a force equal to the resisting force, there would be no net forces on the plates and the plates would maintain a constant velocity between them.

The resisting force is equal to the product of the normal force and a coefficient of kinetic friction. The coefficient of kinetic friction is also a dimensionless quantity, also typically less than one. Furthermore, the coefficient of kinetic friction is usually less than the coefficient of static friction; this is the reason behind the effectiveness of automotive antilock brakes, which can impart a greater stopping force if there is no skidding involved.

Importantly, the resisting force does not depend on the velocity between the plates; as long as there is slipping between the plates, the resisting force depends only on the normal force between the plates.

We may state a second general principle for our clutch: the normal force (i.e., the magnetically-generated force that attracts the plates to each other) determines the torque transmitted from one plate to the other when the plates are slipping.

These two general principles are summarized in Figure 5, which is a plot of the torque transmitted to the proximal end of the drive shaft (vertical axis), versus the torque of the motor (horizontal axis).

If there were no clutch present, and the drive shaft were rotationally attached directly to the motor, the "no slipping" curve in Figure 5 would increase from the origin to the upper right edge of the plot in a 1:1 relationship. In other words, for a clutchless attachment, all of the motor torque is always transmitted to the drive shaft.

At relatively low torques, at which the clutch is engaged and the plates are in contact and do not slip with respect to each other, the 1:1 relationship is seen. In normal use, such as during the spin-up and spin-down portions of the atherectomy cycle, the torques produces by the motor are considered relatively low, so that the clutch remains engaged throughout the procedure. On the plot in Figure 5, this corresponds to the 45-degree branch extending to the right and upward from the origin (labeled "no slipping").

At some particular torque threshold, we want slipping to start, in order to prevent damage to the patient and to the device itself. This threshold occurs at the top-right point of the "no slipping" curve, and is proportional to the normal force. Slipping occurs when the torque of the motor equals or exceeds this threshold value.

When there is slipping between the plates in the clutch, the torque that is transmitted to the drive shaft cannot exceed a particular "slipping" value, regardless of how large the actual torque of the motor is. This limits the maximum torque that can be transmitted to the drive shaft, which also prevents damage to the patient and to the device itself. This "slipping" torque value is also proportional to the normal force, and may be referred to herein as a "residual" torque.

Note that because the kinetic coefficient of friction is generally less than the static coefficient of friction, the two curves intersect as shown in Figure 5, with the "no slipping" portion extending upward at to the right, beyond the intersection point.

In general, the curves in Figure 5 are scalable in proportion to the normal force. If the normal force is doubled, for example, the "no slipping" curve extends twice as far to the top-right, and the "slipping" torque value is doubled. The normal force is controllable during the design phase of the clutch, through the choice of magnetic materials in the plates and the lateral and longitudinal placement of those materials.

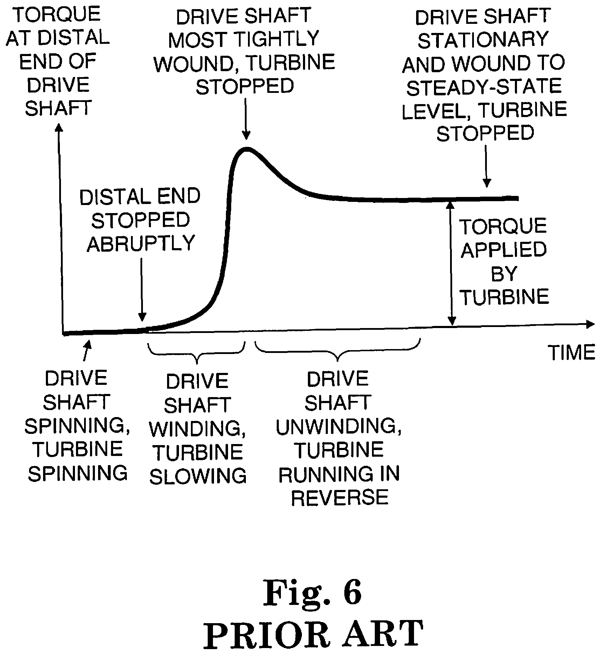

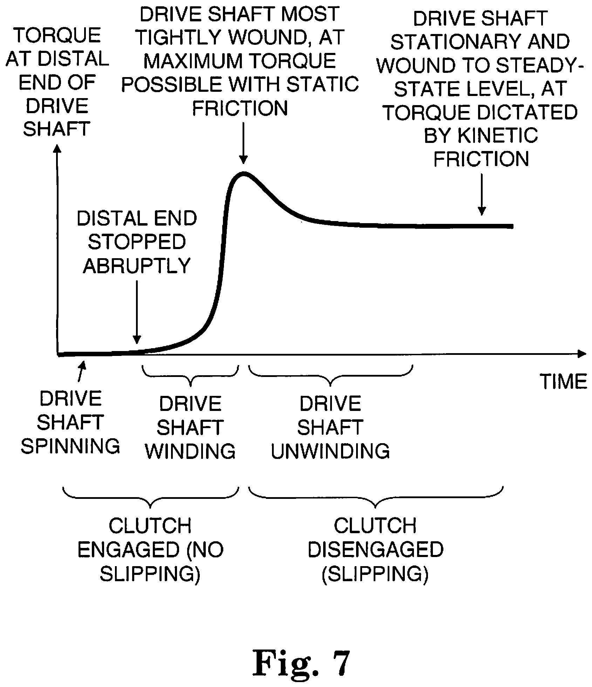

The curves of Figure 5 are plotted as torque versus torque. In order to see how these torques evolve in time when the distal end of the drive shaft is abruptly stopped, two examples are presented in Figure 6 and 7. Figure 6 pertains to a known system, in which the drive shaft is connected to a gas turbine, and does not use a clutch. The rotational inertia of the gas turbine is small enough so that the associated torques do not cause any damage to the patient or to the device. Figure 7 pertains to a system that uses a higher-rotational-inertia motor, such as an electric motor, which uses the clutch to prevent damage. In particular, the peak and steady-state torque values in Figure 7 are chosen to mimic those in Figure 6, which have been determined to be acceptable in practice.

We first turn to Figure 6, which is a plot of torque at the distal end of the drive shaft versus time for a distal-end-stopping event, for a known gas turbine system. The known gas turbine system does not have a clutch.

Initially, both the motor and drive shaft are spinning together. The rotation is assumed to be at a constant rotational speed, so there is no net torque on the distal end of the drive shaft.

Next, the distal end of the drive shaft is stopped abruptly, as would happen if it got stuck or encountered an obstacle in the blood vessel.

Following the abrupt stop, the drive shaft begins to wind up, or rotationally compress. Such a compression is analogous to a linear spring; the more it is compressed, the harder it becomes to impart additional compression. In this phase, the draft shaft essentially "pushes back" rotationally on the motor, and the motor slows down.

There comes a point when all the rotational energy has gone into rotationally compressing the spring, and the spring and motor are stopped at the spring's maximum compression point. At this point, the distal end of the drive shaft experiences its maximum torque.

Following the maximum compression, the drive shaft "springs back" and unwinds a bit. During this unwinding, the motor and the proximal end of the drive shaft run in reverse. In practice, there may be some "ringing" to this curve, as the energy in the system oscillates between kinetic (movement) and potential (rotational compression of the drive shaft). Much of the "ringing" is damped due to friction, and the oscillations become increasingly small as system settles to a stationary steady state. The "ringing" is omitted from Figure 6.

At this steady state, the motor is stopped but is still applying a torque. The drive shaft is also stationary, but is stationary in a rotationally compressed position due to the motor torque.

The entire horizontal axis of Figure 6 may last on the order of milliseconds. The known gas turbine may have a control system that detects when its rotational speed falls below a threshold value or falls to zero and subsequently shuts off the motor. Such a control system may require a particular length of time to react, typically on the order of several seconds. These control systems cannot react directly to portions of the curve of Figure 6, though, because the spike and settling to steady-state typically occurs much more rapidly than the control system can react.

There are two torque values to note on the curve of Figure 6. The first value is the peak value, which occurs when the drive shaft is most tightly wound and the motor is stopped. The second value is the steady-state value. Both of these torque values have been deemed safe for use in the known, gas turbine-driven atherectomy system. As a result, the clutch 40 may be designed to mimic one or both of these safe torque values.

Figure 7 is a plot of torque at the distal end of the drive shaft versus time for a distal-end-stopping event, for the present motor-driven system with the clutch of Figure 3. One difference between Figure 6 and 7 is that for the present clutch design, the motor continues to turn throughout the clutch disengagement; for the known gas turbine of Figure 6, the turbine stops along with the drive shaft. Such a stopping of the present motor is not feasible because of the relatively large rotational inertia of the motor.

Initially, both the motor and drive shaft are spinning together. The rotation is assumed to be at a constant rotational speed, so there is no net torque on the distal end of the drive shaft. The clutch is engaged, and there is no slipping between the plates of the clutch.

Next, the distal end of the drive shaft is stopped abruptly. As with Figure 6, the spiked torque associated with stopping the distal end is omitted from Figure 7.

Following the abrupt stop, the drive shaft begins to wind up, or rotationally compress. In this phase, the draft shaft essentially "pushes back" rotationally on the motor, and the motor may slow down. In practice, this slowing down of the motor may be very slight, because the rotational inertia of the motor may be quite large, especially compared with that of the gas turbine discussed above.

Eventually, as the distal end of the drive shaft remains fixed and the proximal end of the drive shaft continues to wind, there will reach a point when the torque difference between the motor and the proximal end of the drive shaft equals the threshold torque, beyond which the clutch plates start to slip. This threshold point corresponds to the peak of the curve in Figure 7.

One may trace the progress thus far in Figure 5. Initially, while the motor and drive shaft are spinning together, the system as at the origin. After the distal end is stopped, the system rises upward and to the right along the "no slipping" curve. The threshold point, which is the peak of the curve in Figure 7, is at the top-right-most edge of the "no slipping" curve in Figure 5.

Once the plates begin to slip, the clutch becomes disengaged. The motor continues to rotate, along with plate 41 of the clutch 40. The other plate 42, however, rotates more slowly than the plate 41, and eventually stops and unwinds, along with the proximal end of the drive shaft. Once any ringing effects have died off and steady state is reached, the drive shaft is stationary and slightly wound, the proximal end of the drive shaft is stationary, the plate 42 is stationary, the plate 41 remains rotating along with the motor, and rotating plate 42 transmits enough torque to stationary plate 41 to keep the drive shaft slightly wound.

Essentially, the torque transmitted by the clutch 40 in its slipping mode is analogous to the torque of the gas turbine of Figure 6 when the gas turbine is stationary. In fact, during the design phase of the clutch 40, the attractive magnetic normal force between the plates can be set so that the steady-state torque of Figure 7 matches that of Figure 6, since the steady-state torque of the gas turbine has been deemed safe for use. Alternatively, the attractive magnetic normal force between the plates can be set so that the peak torque, i.e., the threshold torque value at which the plates begin to slip (the peak in Figure 7), matches that of Figure 6. As a further alternative, both the peak and steady-state torque values can be met by texturing one or both surfaces of the clutch, adjusting the diameter of the contact surfaces, and/or adjusting the materials on the opposing faces in the clutch.

Although the plates 41 and 42 are drawn in Figure 3 as being coaxial and circular, other suitable shapes and orientations may be used. One or both surfaces may optionally be textured, which can adjust the surface area in contact and may affect the frictional performance of the interface. In addition, the plates 41 and 42 may optionally be curved, and may have mating curvatures that fit together. For instance, one plate may be convex with a particular radius of curvature, and the other plate may be concave with the same radius of curvature.

The description of the invention and its applications as set forth herein is illustrative and is not intended to limit the scope of the invention. Variations and modifications of the embodiments disclosed herein are possible, and practical alternatives to and equivalents of the various elements of the embodiments would be understood to those of ordinary skill in the art upon study of this patent document. These and other variations and modifications of the embodiments disclosed herein may be made without departing from the scope of the invention as defined by the claims.

## Claims

1. A rotational atherectomy system, comprising:
an elongated, rotatable, flexible and disposable drive shaft (20) having a distal end for insertion into a vasculature of a patient and having a proximal end opposite the distal end remaining outside the vasculature of the patient;
a motor (30) capable of rotating the disposable drive shaft (20); and
a clutch (40) having a characteristic threshold torque, the clutch (40) comprising:
a magnetic motor plate (41) rotationally connected to the motor (30); and
a disposable magnetic drive shaft plate (42) rotationally connected to the proximal end of the drive shaft (20), the magnetic motor plate (41) comprising a contact face and the disposable magnetic drive shaft plate (42) comprising an opposing contact face, the contact face and the opposing contact face being planar, coaxial and comprising an interface therebetween when in contact with each other, the magnetic motor plate (41) and the disposable magnetic drive shaft plate (42) being disposed directly longitudinally adjacent to each other, and the magnetic motor plate (41) and the disposable magnetic drive shaft plate (42) being held in fictional contact with one another longitudinally by a magnetic attractive force between the magnetic motor plate (41) and the disposable magnetic drive shaft plate (42), the magnetic attractive force being normal to the contact face of the magnetic motor plate (41), normal to the opposing contact face of the disposable magnetic drive shaft plate (42) and normal to the interface therebetween, and wherein the disposable magnetic drive shaft plate (42) is detachable from the magnetic motor plate (41);
wherein torques between the motor (30) and the drive shaft (20) less than the threshold torque:
are transmitted completely between the contact face of the magnetic motor plate (41) and the opposing contact face of the disposable magnetic drive shaft plate (42), which remain held together rotationally by static friction at the interface;
wherein torques between the motor (30) and the drive shaft (20) greater than the threshold torque:
cause the contact face of the magnetic motor plate (41) and the opposing contact face of the disposable magnetic drive shaft plate (42) to slip rotationally past each other at the interface; and
cause a residual torque to be transmitted between the motor (30) and the drive shaft (20), the residual torque being less than the threshold torque and being determined by a kinetic coefficient of friction between the contact face of the magnetic motor plate (41) and the opposing contact face of the disposable magnetic drive shaft plate (42); and
wherein the threshold torque is directly proportional to the magnetic attractive force and a static coefficient of friction between the magnetic motor plate (41) and the disposable magnetic drive shaft plate (42), and represents the maximum torque at which static friction can hold the magnetic motor plate (41) and the disposable magnetic drive shaft plate (42) together rotationally at the interface between the contact face of the magnetic motor plate (41) and the opposing contact face of the disposable magnetic drive shaft plate (42),

2. The system of claim 1, wherein the magnetic normal force decreases as lateral and/or longitudinal spacing between magnetic particles in the magnetic motor plate (41) and in the disposable magnetic drive shaft plate (42) increases.

3. The system of claim 2, wherein the increase in lateral and/or longitudinal spacing between the magnetic particles is achieved by coating the magnetic particles with a non-magnetic layer.

4. The system of claim 1, wherein the magnetic normal force increases as lateral and/or longitudinal spacing between magnetic particles in the magnetic motor plate (41) and/or in the disposable magnetic drive plate (42) decreases.

5. The system of claim 1, wherein the residual torque is directly proportional to the magnetic attractive force.

6. The system of claim 5, wherein the residual torque is independent of the values of the torque between the motor (30) and the drive shaft (20).

7. The system of claim 1, wherein the contact face of the magnetic motor plate (41) and the opposing contact face of the disposable magnetic drive shaft plate (42) have mated curvatures.

## Patentansprüche

1. Ein Rotationsatherektomiesystem, aufweisend:
eine langgestreckte, drehbare, flexible Einweg-Antriebswelle (20), welche ein distales Ende zum Einführen in ein Gefäßsystem eines Patienten und ein proximales Ende hat, das zum distalen Ende entgegengesetzt ist und außerhalb des Gefäßsystems des Patienten verbleibt,
einen Motor (30), welcher in der Lage ist, die Einweg-Antriebswelle (20) zu drehen, und
eine Kupplung (40), welche ein charakteristisches Schwellendrehmoment hat, wobei die Kupplung (40) aufweist:
eine Magnet-Rotorplatte (41), welche mit dem Motor (30) drehbar verbunden ist, und
eine Magnet-Einweg-Antriebswellenplatte (42), welche mit dem proximalen Ende der Antriebswelle (20) drehbar verbunden ist, wobei die Magnet-Motorplatte (41) eine Kontaktfläche aufweist und die Magnet-Einweg-Antriebswellenplatte (42) eine entgegengesetzte Kontaktfläche aufweist, die Kontaktfläche und die entgegengesetzte Kontaktfläche planar und koaxial sind und eine Schnittstelle dazwischen aufweisen, wenn sie sich miteinander in Kontakt befinden, die Magnet-Motorplatte (41) und die Magnet-Einweg-Antriebswellenplatte (42) längs direkt zueinander benachbart angeordnet sind, und die Magnet-Motorplatte (41) und die Magnet-Einweg-Antriebswellenplatte (42) durch eine magnetische Anziehungskraft zwischen der Magnet-Motorplatte (41) und der Magnet-Einweg-Antriebswellenplatte (42) längs miteinander in Reibkontakt gehalten werden, wobei die magnetische Anziehungskraft zur Kontaktfläche der Magnet-Motorplatte (41) senkrecht ist, zur entgegengesetzten Kontaktfläche der Magnet-Einweg-Antriebswellenplatte (42) senkrecht ist und zur Schnittstelle dazwischen senkrecht ist, und wobei die Magnet-Einweg-Antriebswellenplatte (42) von der Magnet-Motorplatte (41) lösbar ist,
wobei Drehmomente zwischen dem Motor (30) und der Antriebswelle (20), welche geringer sind als das Schwellendrehmoment:
vollständig zwischen der Kontaktfläche der Magnet-Motorplatte (41) und der entgegengesetzten Kontaktfläche der Magnet-Einweg-Antriebswellenplatte (42) übertragen werden, welche an der Schnittstelle durch Haftreibung rotationsmäßig zusammengehalten werden,
wobei Drehmomente zwischen dem Motor (30) und der Antriebswelle (20), welche größer sind als das Schwellendrehmoment:
verursachen, dass die Kontaktfläche der Magnet-Motorplatte (41) und die entgegengesetzte Kontaktfläche der Magnet-Einweg-Antriebswellenplatte (42) an der Schnittstelle rotationsmäßig aneinander vorbeirutschen, und
verursachen, dass ein Restdrehmoment zwischen dem Motor (30) und der Antriebswelle (20) übertragen wird, wobei das Restdrehmoment geringer ist als das Schwellendrehmoment und durch einen Reibungskoeffizienten zwischen der Kontaktfläche der Magnet-Motorplatte (41) und der entgegengesetzten Kontaktfläche der Magnet-Einweg-Antriebswellenplatte (42) festgelegt ist, und
wobei das Schwellendrehmoment zur magnetischen Anziehungskraft und zu einem Reibungskoeffizienten zwischen der Magnet-Motorplatte (41) und der Magnet-Einweg-Antriebswellenplatte (42) direkt proportional ist und das Maximaldrehmoment repräsentiert, bei welchem Haftreibung die Magnet-Motorplatte (41) und die Magnet-Einweg-Antriebswellenplatte (42) an der Schnittstelle zwischen der Kontaktfläche der Magnet-Motorplatte (41) und der entgegengesetzten Kontaktfläche der Magnet-Einweg-Antriebswellenplatte (42) rotationsmäßig zusammenhalten kann.

2. Das System gemäß Anspruch 1, wobei die magnetische Normalkraft mit steigendem Lateral- und/oder Longitudinalabstand zwischen Magnetpartikeln in der Magnet-Motorplatte (41) und in der Magnet-Einweg-Antriebswellenplatte (42) abnimmt.

3. Das System gemäß Anspruch 2, wobei die Steigerung im Lateral- und/oder Longitudinalabstand zwischen den Magnetpartikeln erzielt wird durch Beschichten der Magnetpartikel mit einer unmagnetischen Schicht.

4. Das System gemäß Anspruch 1, wobei die magnetische Normalkraft mit sinkendem Lateral- und/oder Longitudinalabstand zwischen den Magnetpartikeln in der Magnet-Motorplatte (41) und/oder in der Magnet-Einweg-Antriebsplatte (42) zunimmt.

5. Das System gemäß Anspruch 1, wobei das Restdrehmoment zur magnetischen Anziehungskraft direkt proportional ist.

6. Das System gemäß Anspruch 5, wobei das Restdrehmoment von den Werten des Drehmoments zwischen dem Motor (30) und der Antriebswelle (20) unabhängig ist.

7. Das System gemäß Anspruch 1, wobei die Kontaktfläche der Magnet-Motorplatte (41) und die entgegengesetzte Kontaktfläche der Magnet-Einweg-Antriebswellenplatte (42) zueinander passende Krümmungen haben.

## Revendications

1. Système d'athérectomie rotatif comprenant :
un arbre d'entraînement allongé, rotatif, flexible et jetable (20) ayant une extrémité distale pour l'insertion dans un système vasculaire d'un patient et ayant une extrémité proximale opposée à l'extrémité distale restant à l'extérieur du système vasculaire du patient ;
un moteur (30) pouvant faire tourner l'arbre d'entraînement jetable (20) ; et
un embrayage (40) ayant un couple de seuil caractéristique, l'embrayage (40) comprenant :
une plaque de moteur magnétique (41) raccordée, en rotation, au moteur (30) ; et
une plaque d'arbre d'entraînement magnétique jetable (42) raccordée, en rotation, à l'extrémité proximale de l'arbre d'entraînement (20), la plaque de moteur magnétique (41) comprenant une face de contact et la plaque d'arbre d'entraînement magnétique jetable (42) comprenant une face de contact opposée, la face de contact et la face de contact opposée étant planaires, coaxiales et comprenant une interface entre elles lorsqu'elles sont en contact l'une avec l'autre, la plaque de moteur magnétique (41) et la plaque d'arbre d'entraînement magnétique jetable (42) étant disposées directement longitudinalement de manière adjacente entre elles, et la plaque de moteur magnétique (41) et la plaque d'arbre d'entraînement magnétique jetable (42) étant maintenues en contact de friction l'une par rapport à l'autre longitudinalement par une force d'attraction magnétique entre la plaque de moteur magnétique (41) et la plaque d'arbre d'entraînement magnétique jetable (42), la force d'attraction magnétique étant normale par rapport à la face de contact de la plaque de moteur magnétique (41), normale par rapport à la face de contact opposée de la plaque d'arbre d'entraînement magnétique jetable (42) et normale par rapport à l'interface entre elles, et dans lequel la plaque d'arbre d'entraînement magnétique jetable (42) peut être détachée de la plaque de moteur magnétique (41) ;
dans lequel les couples entre le moteur (30) et l'arbre d'entraînement (20) inférieurs au couple de seuil :
sont complètement transmis entre la face de contact de la plaque de moteur magnétique (41) et la face de contact opposée de la plaque d'arbre d'entraînement magnétique jetable (42), qui restent maintenues ensemble, en rotation, par la friction statique au niveau de l'interface ;
dans lequel les couples entre le moteur (30) et l'arbre d'entraînement (20) supérieurs au couple de seuil :
amènent la face de contact de la plaque de moteur magnétique (41) et la face de contact opposée de la plaque d'arbre d'entraînement magnétique jetable (42) à glisser, en rotation, l'une au-delà de l'autre au niveau de l'interface ; et
amènent un couple résiduel à être transmis entre le moteur (30) et l'arbre d'entraînement (20), le couple résiduel étant inférieur au couple de seuil et étant déterminé par le coefficient de friction cinétique entre la face de contact de la plaque de moteur magnétique (41) et la face de contact opposée de la plaque d'arbre d'entraînement magnétique jetable (42) ; et
dans lequel le couple de seuil est directement proportionnel à la force d'attraction magnétique et à un coefficient de friction statique entre la plaque de moteur magnétique (41) et la plaque d'arbre d'entraînement magnétique jetable (42), et représente le couple maximum auquel la friction statique peut maintenir la plaque de moteur magnétique (41) et la plaque d'arbre d'entraînement magnétique jetable (42) ensemble, en rotation, au niveau de l'interface entre la face de contact de la plaque de moteur magnétique (41) et la face de contact opposée de la plaque d'arbre d'entraînement magnétique jetable (42).

2. Système selon la revendication 1, dans lequel la force magnétique normale diminue au fur et à mesure que l'espacement latéral et/ou longitudinal entre les particules magnétiques dans la plaque de moteur magnétique (41) et dans la plaque d'arbre d'entraînement magnétique jetable (42) augmente.

3. Système selon la revendication 2, dans lequel l'augmentation dans l'espacement latéral et/ou longitudinal entre les particules magnétiques est obtenue en recouvrant les particules magnétiques avec une couche non magnétique.

4. Système selon la revendication 1, dans lequel la force magnétique normale augmente au fur et à mesure que l'espacement latéral et/ou longitudinal entre les particules magnétiques dans la plaque de moteur magnétique (41) et/ou dans la plaque d'entraînement magnétique jetable (42) diminue.

5. Système selon la revendication 1, dans lequel le couple résiduel est directement proportionnel à la force d'attraction magnétique.

6. Système selon la revendication 5, dans lequel le couple résiduel est indépendant des valeurs du couple entre le moteur (30) et l'arbre d'entraînement (20).

7. Système selon la revendication 1, dans lequel la face de contact de la plaque de moteur magnétique (41) et la face de contact opposée de la plaque d'arbre d'entraînement magnétique jetable (42) ont des courbures couplées.
